# EUROPEAN PATENT APPLICATION

(11) **EP 1 420 353 A2**
(43) Date of publication of application: **19.05.2004**
(21) Application number: 03022858.9
(22) Date of filing: 08.10.2003
(51) Int. Cl.: G06F 19/00

(54) **Medical service assisting system**

(30) Priority: 06.11.2002 JP 2002322216
(71) Applicant: Olympus Corporation, Tokyo 151-0072 (JP)
(72) Inventor: Ryoichi, Hosoya, Ichikawa-shi Chiba 272-0021 (JP)
(74) Representative: von Hellfeld, Axel, Dr. Dipl.-Phys.

(57) **Abstract**

In an examination conduct information storing unit, examination conduct information, which is information occurring by conducting an examination in a medical institution, is stored, each time the examination is conducted. A totaling unit calculates a total amount of time required for conducting the examination, which is indicated by the examination conduct information stored in the examination conduct information storing unit.

## Description

### Cross Reference to related application

The application is based upon and claims the benefit of priority from the prior Japanese Application No. 2002-322216, filed Nov. 6, 2002, the entire contents of which are incorporated herein by reference.

### Background of the Invention

### Field of the Invention

The present invention relates to a technology for assisting in a medical service, and more particularly, to a technology for assisting in administrative operations of each type of an examination.

### Description of the Related Art

As one of service assistances rendered by a system assisting in a service on a medical scene, assistance in administrative operations of each type of examination exists. This is intended to reserve an examination date and time based on an examination request issued, for example, from a terminal in each department connected to a hospital-wide network and draw up an examination schedule, and to store a record of the conduct status of an examination and its result.

For example, Japanese Patent Publication No. 2002-73615 discloses the invention of a medical image filing system assisting in such administrative operations of an examination. With such a conventional system, setting of a required time, which is expected for an examination when a reservation is made, can be controlled. Here, a time required for an examination, and the number of staff members such as a doctor, a nurse, etc., who are responsible for the examination, are fixedly preplanned according to prediction based on examination experiences in the past.

### Summary of the Invention

A medical service assisting system as one embodiment of the present invention is configured to comprise an examination conduct information storing unit storing examination conduct information, which is information occurring by conducting an examination in a medical institution, each time the examination is conducted, and a totaling unit calculating a total amount of time required for conducting the examination, which is indicated by the examination conduct information stored in the examination conduct information storing unit.

In the above described configuration, preferably, the system may be configured to further comprise an obtaining unit obtaining an input of the examination conduct information, and a displaying unit displaying a result of the total.

Additionally, preferably, the total amount of time may be a total for each doctor who is responsible for the examination, for each number of assisting persons who assist in the examination, or for each examination technique in the examination.

Furthermore, preferably, the totaling unit may calculate an average of the required time as a result of the total.

Still further, preferably, the system may comprise a display controlling unit graphing the result of the total, and causing the displaying unit to display the result.

### Brief Description of the Drawings

The present invention will be more apparent from the following detailed description when the accompanying drawings are referenced.
Fig. 1 shows a principle configuration of a system implementing the present invention;
Fig. 2 shows a specific configuration of the system implementing the present invention;
Fig. 3 shows the configurations of an examination information inputting/searching terminal, and a data server;
Fig. 4 exemplifies part of a data structure of examination conduct information;
Fig. 5 shows a work flow of administrative operations of an endoscopic examination conducted with the system shown in Fig. 2;
Fig. 6 is a flowchart showing the contents of a registration process;
Fig. 7 exemplifies an examination information input screen;
Figs. 8A and 8B are flowcharts showing the contents of a totaling process;
Fig. 9 exemplifies a graph of examination times against respective doctors;
Fig. 10 exemplifies a graph of examination times against the numbers of nurses;
Fig. 11 exemplifies a graph of examination times against examination techniques; and
Fig. 12 exemplifies a storage medium from which a recorded control program can be read by a computer.

### Description of the Preferred Embodiments

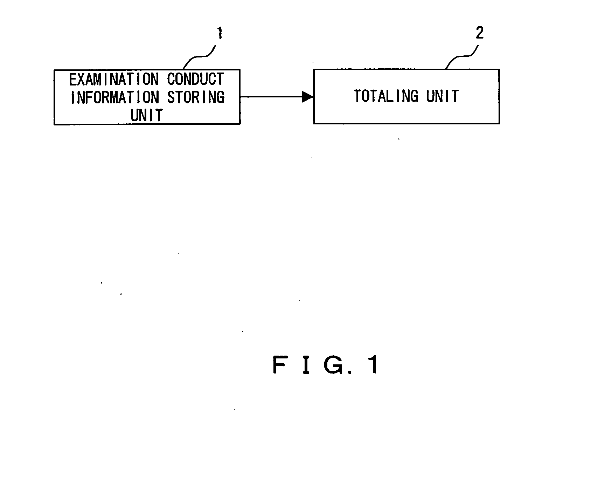

Firstly, Fig. 1 is explained. This figure shows a principle configuration of a medical service assisting system implementing the present invention.

An examination conduct information storing unit 1 stores examination conduct information, which is information occurring by conducting an examination in a medical institution, each time the examination is conducted.

A totaling unit 2 calculates total amount of time required for conducting the examination, which is indicated by the examination conduct information stored in the examination conduct information storing unit 1.

With this configuration, a prediction of a required time, which is expected for an examination newly reserved, can be made by using a result of the total amount of time required for the same examination conducted in the past, which is obtained by the totaling unit 2.

The above described medical service assisting system implementing the present invention may be configured to further comprise an obtaining unit obtaining an input of the above described examination conduct information, and a displaying unit displaying a result of the above described total.

With this configuration, examination conduct information about a newly conducted examination is input, whereby the examination conduct information can be reflected on a prediction of a required time, which is expected for an examination reserved later. As a result, a more probable prediction can be made.

Additionally, in the above described medical service assisting system implementing the present invention, the total calculated by the totaling unit 2 may be a total for each doctor who is responsible for an examination, for each number of assisting persons who assist in the examination, or for each technique of the examination.

With this configuration, a prediction of a required time, which is expected for an examination newly reserved, can be made based on a doctor who is responsible for the examination, the number of assisting persons who assist in the examination, or a technique of the examination, so that a more probable prediction can be made.

Additionally, in the above described medical service assisting system implementing the present invention, the totaling unit 2 may calculate an average amount of time required for conducting an examination as a result of the total.

With this configuration, a prediction of a required time, which is expected for an examination newly reserved, can be made based on the average amount of time a technique of the examination took to complete in the past, whereby a more probable prediction can be made.

Additionally, in this configuration, a display controlling unit graphing a result of the total calculated by the totaling unit 2, and causing the displaying unit to display the result may be further comprised.

With this configuration, the result of the total calculated by the totaling unit 2 is visually provided by means of a graph, so that the result of the total can be grasped with ease.

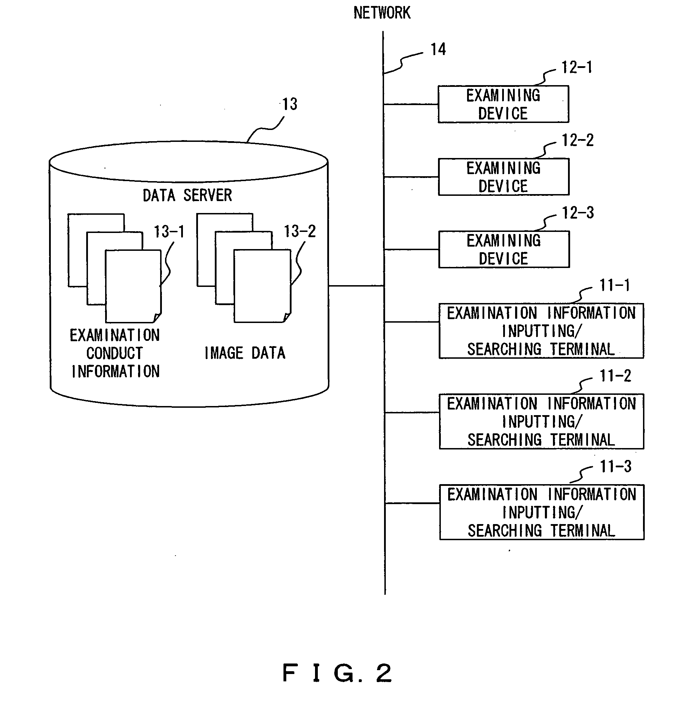

Next, Fig. 2 is explained. This figure shows a specific configuration of the system implementing the present invention. The system shown in this figure is a system assisting in administrative operations of an endoscopic examination in an endoscopic examination department in a hospital, and comprises functions such as reservation management of an endoscopic examination, recording management of the conduct status of an endoscopic examination, storage management of a shot image, creation/management of an examination result report, etc.

The system shown in Fig. 2 is configured by connecting examination information inputting/searching terminals 11-1, 11-2, and 11-3, examining devices 12-1, 12-2, and 12-3, and a data server 13 respectively to a network 14. Various types of data can be mutually exchanged among these appliances via the network 14.

All of the examination information inputting/searching terminals 11-1, 11-2, and 11-3 are terminals used by users of this system, and are used to reserve an endoscopic examination or verify the reserved contents, to input or browse the conduct status of an endoscopic examination, or to create or browse an examination result report.

All of the examining devices 12-1, 12-2, and 12-3 are electronic endscopes, and have a function for outputting to the network 14 image data that represents a shot image, and information about shooting of that image, such as shooting date and time, a used instrument name, etc.

The data server 13 is a data server that altogether stores various types of data such as examination conduct information 13-1 obtained by the examination information inputting/searching terminal 11-1, 11-2, or 11-3, image data 13-2 output from the examining device 12-1, 12-2, or 12-3, and the like, and provides these data by a request made from the examination information inputting/searching terminal 11-1, 11-2, or 11-3.

Although the three examination information inputting/searching terminals 11-1, 11-2, and 11-3, and the three examining devices 12-1, 12-2, and 12-3 are shown in Fig. 2, the numbers of these terminals and devices may be arbitrary.

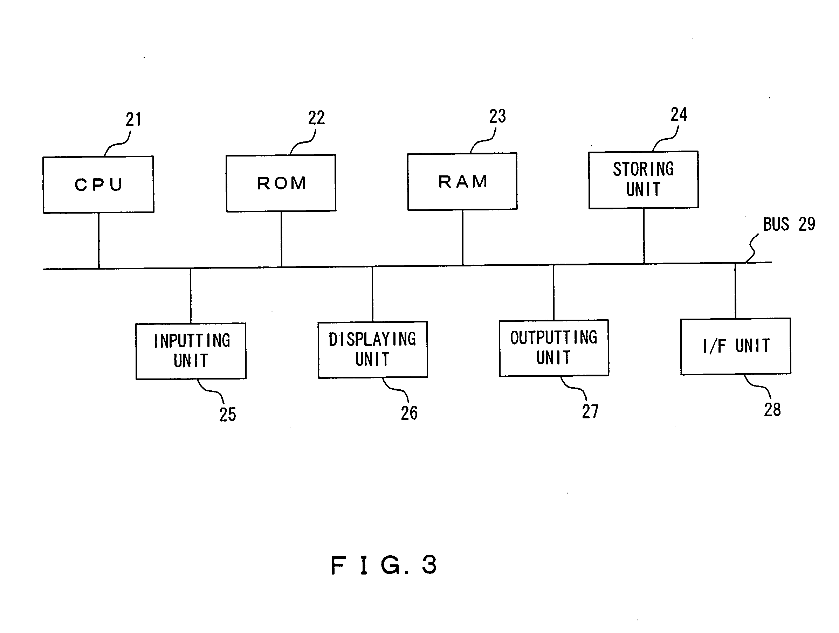

Next, Fig. 3 is explained. This figure shows the configuration of the examination information inputting/searching terminals 11-1, 11-2, and 11-3, and the data server 13, which are shown in Fig. 2.

The device shown in Fig. 3 is configured by interconnecting a CPU 21, a ROM 22, a RAM 23, a storing unit 24, an inputting unit 25, a displaying unit 26, an outputting unit 27, and an I/F unit 28 via a bus 29, and these memories and units can mutually exchange data under the management of the CPU 21.

The CPU (Central Processing Unit) 21 is a central processing unit that governs the operation control of the entire device shown in Fig. 3.

The ROM (Read Only Memory) 22 is a memory in which a basic control program executed by the CPU 21 is prestored. The CPU 21 executes this basic control program when this device is started up, whereby the basic control of the operations of the entire device is performed by the CPU 21.

The RAM (Random Access Memory) 23 is a memory that is used as a working memory when the CPU 21 executes each type of a control program, and also serves as a main memory used as a temporary storage area of each type of data depending on need.

The storing unit 24 is configured by comprising, for example, an HDD (Hard Disk Drive). When the device shown in Fig. 3 is used as the examination information inputting/searching terminal 11-1, 11-2, or 11-3, a control program for causing the CPU 21 to perform a registration process and a totaling process, which will be described later, is prestored in the storing unit 24. Additionally, when the device shown in Fig. 3 is used as the data server 13, the storing unit 24 serves as a data storing device which stores various types of data such as the examination conduct information 13-1, image data 13-2, etc. Furthermore, at this time, the storing unit 24 prestores a control program for causing the CPU 21 to perform a data operation process for this database according to an instruction of each type of data operation transmitted from the examination information inputting/searching terminal 11-1, 11-2, or 11-3.

The inputting unit 25 is intended to receive an external input, and to pass its contents to the CPU 21, and comprises an inputting device, such as a keyboard, a mouse, etc., which receives an instruction from an operator who operates this device. Additionally, the inputting unit 25 is configured by comprising, depending on need, a reading device of a portable storage medium such as an FD (Flexible Disk), a CD-ROM (Compact Disc -ROM), a DVD-ROM (Digital Versatile Disc-ROM), an MO (Magneto-Optics) disk, etc.

The displaying unit 26 is intended to display various types of information according to an instruction from the CPU 21, and is configured by comprising, for example, a CRT (Cathode Ray Tube), an LCD (Liquid Crystal Display), etc.

The outputting unit 27 is intended to output various types of information according to an instruction from the CPU 21, and is, for example, a printer device for printing contents of a display made on the displaying unit 26 on a sheet unchanged, or the like.

The I/F (interface) unit 28 makes communication management when data is exchanged with another device by connecting this device to the network 14 shown in Fig. 2.

The device shown in Fig. 3 is merely a device normally comprised by a computer system having a standard hardware configuration. Therefore, the examination information inputting/searching terminal 11-1, 11-2, or 11-3, or the data server 13 can be also configured by diverting such a computer system.

For ease of explanation, the following explanation assumes that 11-1 and 13-1, which are shown in Fig. 2, are used respectively as an examination information inputting/searching terminal and an examining device, unless otherwise noted.

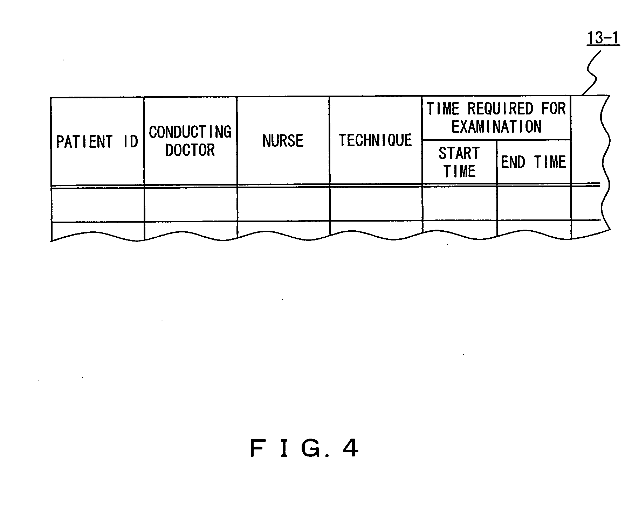

Next, Fig. 4 is explained. This figure exemplifies part of a data structure of the examination conduct information 13-1 stored in the image and data server 13.

For the examination conduct information 13-1, one file is created for each examination day in the data server 13. As shown in Fig. 4, in each record in a file, fields for storing various items of information such as a number of a "patient ID" for identifying a patient, a name of a "conducting doctor" who is responsible for an examination, a name of a "nurse" who is an assisting person assisting in conducting the examination, contents of a "technique" of the examination, and "start time" and "end time" of the examination as information indicating the conduct time of the examination, and the like are provided. Here, a record is created for each "technique" when a plurality of "techniques" are conducted in one examination. Additionally, a plurality of names can be stored in the "nurse" field in one record.

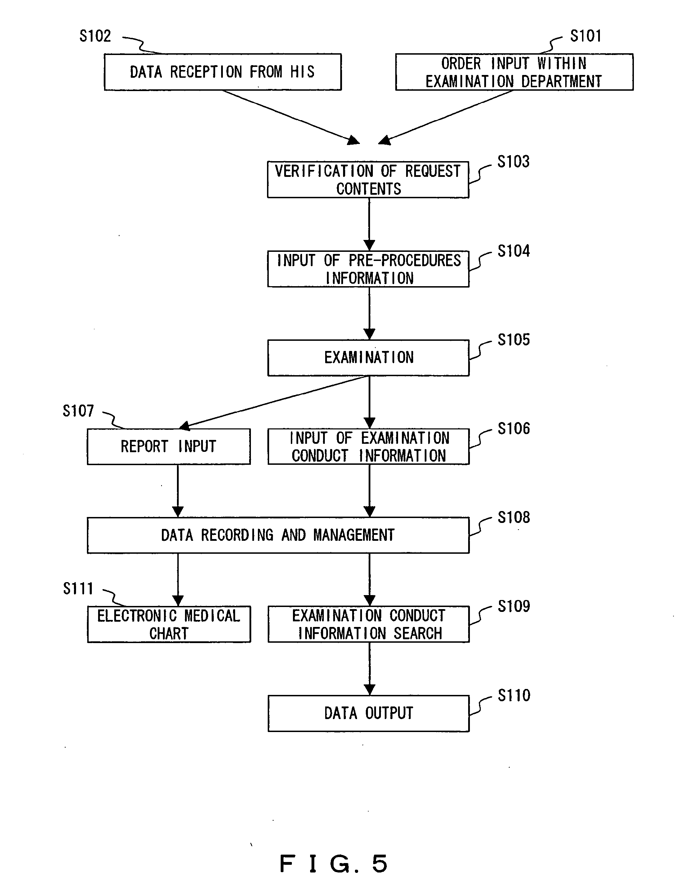

Next, Fig. 5 is explained. This figure shows a work flow of administrative operations of an endoscopic examination conducted by using the system shown in Fig. 2.

Firstly, in S101, the inputting unit 25 of the examination information inputting/searching terminal 11-1 is operated, and an examination order (reservation) is input. This examination order may be generated in a way such that the examination information inputting/searching terminal 11-1 receives data from an appliance such as an HIS (Hospital Information Server), etc., which is connected to the network 14, is not shown in Fig. 2, and governs the information management of the entire hospital as indicated by S102.

In S103, contents of a request in the examination order that is obtained with the operation in S101 or S102 is displayed on the displaying unit 26 comprised by the examination information inputting/searching terminal 11-1, and verification of the contents of the request by a responsible person in an examination department, settings of date and time when the examination is conducted, responsible staff members, and the like are made.

In S104, pre-procedures required prior to the endoscopic examination are performed. At that time, the inputting unit 25 of the examination information inputting/searching terminal 11-1 is operated, and information about the pre-procedures, such as date and time when the pre-procedures are performed, a person who performs the pre-procedures, a drug used for the pre-procedures, and the like are input.

In S105, the endoscopic examination is conducted by using the examining device 12-1, and an image is shot.

In S106, the inputting unit 25 of the examination information inputting/searching terminal 11-1 is operated, and information about the examination conducted in S105, such as a technique conducted, a medical expense item added, a person who conducted the examination, a drug and an instrument used for the examination, and the like are input.

Additionally, in S107, the inputting unit 25 of the examination information inputting/searching terminal 11-1 is operated, and a report that summarizes findings of a doctor about the result of the examination conducted in S105, and the like is input.

In S108, the image obtained in S105 is stored in the data server 13 as the image data 13-2, and at the same time, the information obtained by the examination information inputting/searching terminal 11-1 with the operations up to S107 are altogether stored in the data server 13 as the examination conduct information 13-1.

Thereafter, in S109, when an instruction to search for the examination conduct information 13-1 is issued to the examination information inputting/searching terminal 11-1, a search request is given from the examination information inputting/searching terminal 11-1 to the data server 13, and the examination conduct information 13-1 that meets a condition for that instruction is searched. The examination information inputting/searching terminal 11-1 that obtains a result of this search graphs the result of this search, and displays the result on the displaying unit 26 in S110.

Additionally, in S111, when an instruction to output an electronic medical chart is issued to the examination information inputting/searching terminal 11-1, the examination conduct information 13-1 associated with that instruction is transmitted from the data server 13 to the examination information inputting/searching terminal 11-1, which outputs this information by using the displaying unit 26 or the outputting unit 27.

The above described administrative operations of the endoscopic examination for the above described work flow are performed with the system shown in Fig. 2.

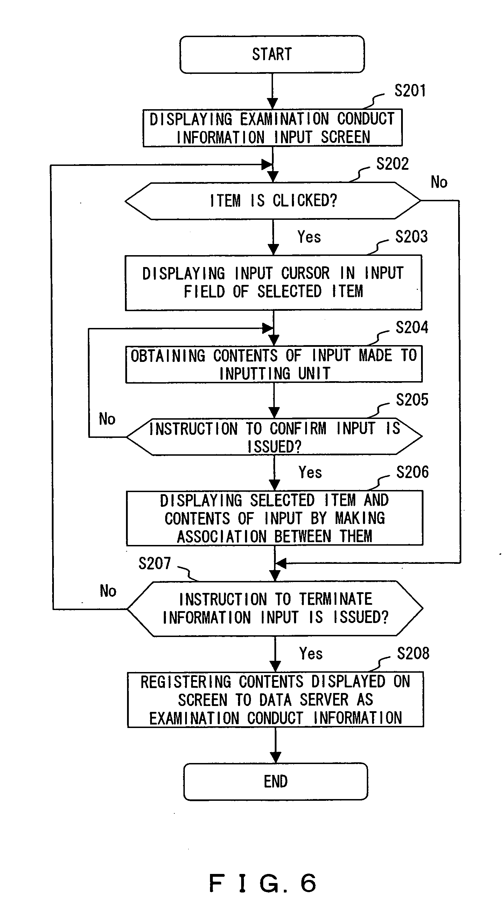

Next, Fig. 6 is explained. This figure is a flowchart showing the contents of a registration process for registering the examination conduct information 13-1 to the data server 13. This process is a process performed in common in the operations of S104, S106, etc. in the work flow shown in Fig. 5. Process steps that are not particularly noted in the subsequent explanation among respective process steps shown in this figure are performed by the CPU 21 of the examination information inputting/searching terminal 11-1. The CPU 21 implements these processes by reading and executing the control program stored in the storing unit 24 of the examination information inputting/searching terminal 11-1.

This registration process shown in Fig. 6 is started when an instruction to input examination information, which is issued with an operation for the inputting unit 25 of the examination information inputting/searching terminal 11-1, is detected.

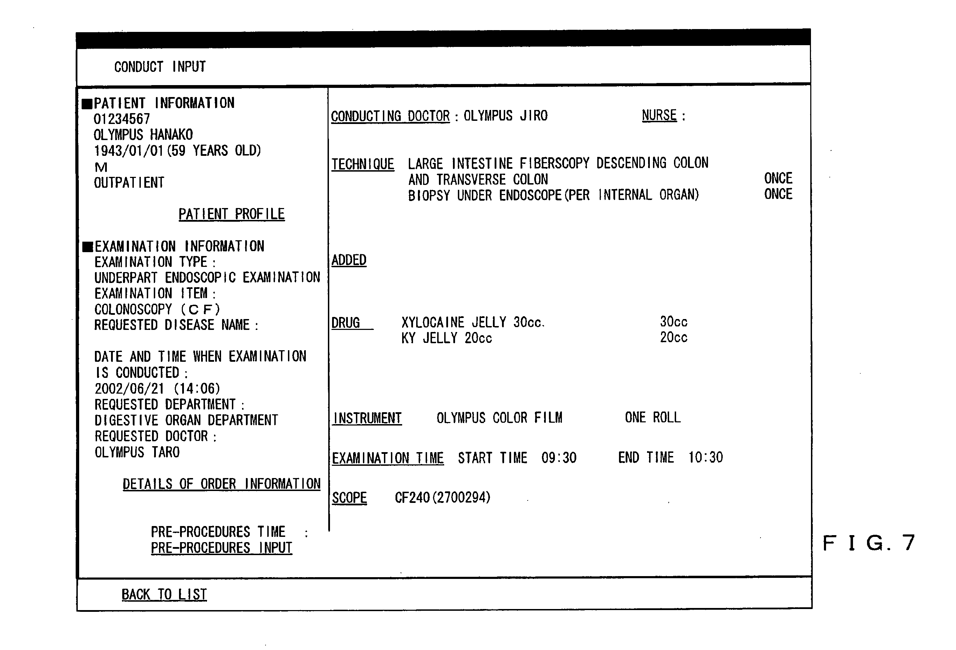

Firstly, in S201, a process for causing the displaying unit 26 of the examination information inputting/searching terminal 11-1 to display an examination information input screen is performed. This examination information input screen is exemplified in Fig. 7.

On the screen shown in Fig. 7, patient information and examination information are displayed on its left side. The patient information is information about an ID (Identification) number preassigned to each patient, a patient name, a birth date/age, sex, type (inpatient or outpatient, and the like), etc. Furthermore, the examination information is information about requested contents of an examination, such as an examination type, an examination item, a disease name at the time of request, scheduled date and time when the examination is conducted, a requested department of the examination, a requested doctor, etc.

Additionally, the right side of Fig. 7 is a screen on which contents of respective information items to be registered to the data server 13 as the examination conduct information 13-1 with this registration process are displayed. A name of a "conducting doctor" who is responsible for an examination, a name of a "nurse" who is responsible for the examination, contents of a "technique" of the examination, a medical expense item to be "added", a "drug" used for pre-procedures or the examination, an "instrument" used for the examination, "start time" and "end time" of the examination, and contents of information for identifying an endoscope ("scope") used for the examination are arranged and displayed by item. This figure indicates the state where no inputs are made to the "nurse" and "added" items, namely, the state where there are no targets for the "nurse" and "added" items.

Turning back to the explanation of Fig. 6.

In S202, it is determined whether or not any of the respective items of the examination conduct information 13-1 is clicked, namely, whether or not a position indicating cursor, which is overlaid and displayed on the examination information input screen displayed on the displaying unit 26, is moved according to an operation for a mouse, etc. comprised by the inputting unit 25, and a mouse button, etc. is operated when the position indicating cursor is placed on a character string that indicates each underscored item on the right side of Fig. 7. If a result of this determination is "Yes", the process proceeds to S203. If the result of this determination is "No", the process proceeds to S207.

In S203, an input cursor which indicates a character input position is displayed in an input field for an item selected on the examination information input screen, namely, the item clicked with the process of the preceding step.

In S204, contents of an input of the character string which corresponds to the operation for the inputting unit 25 of the examination information inputting/searching terminal 11-1 is obtained.

In S205, it is determined whether or not the contents of the operation for the inputting unit 25 of the examination information inputting/searching terminal 11-1 is an instruction to confirm the input of the character string. If a result of this determination is "Yes", the process proceeds to S206. If the result of this determination is "No", the process returns to S204, and the process for obtaining an input of a character string is continued.

In S206, the character string obtained with the process of S204 is corresponded to the aforementioned selected item, and displayed on the examination information input screen.

In S207, it is determined whether or not an instruction to terminate the input of the examination information is issued with an operation for the inputting unit 25 of the examination information inputting/searching terminal 11-1. If a result of this determination is "Yes", the process proceeds to S208. If the result of this determination is "No", the process returns to S202, and the above described process is repeated.

In S208, data that becomes the examination conduct information 13-1 is created based on the contents of the display made on the examination information input screen displayed on the displaying unit 26, and the created data is addressed to the data server 13, and transmitted to the network 14. When the data server 13 receives this data, the CPU 21 that is executing the control program newly creates a record of the examination conduct information 13-1, in which this data is indicated in the respective fields, and stores the record in the storing unit 24 of the data server 13.

The process up to this point is the registration process.

In this process, a character input made to the inputting unit 25 is obtained to acquire the contents of the respective items. Alternatively, the contents of the respective items may be obtained by displaying a list of words and phrases, which are assumed as the contents of the items, on the displaying unit, and by selecting suitable words and phrases from the list. By doing so, a workload imposed on an operator of the examination information inputting/searching terminal 11-1 in order to input the contents of the respective items is reduced.

Furthermore, contents such as an "examination time", a "scope", etc., which are indicated on the examination information input screen, may be automatically obtained from the examining device 12 via the network 14. Also by doing so, the workload of the operations for inputting these items, which is imposed on the operator of the examination information inputting/searching terminal 11-1, is reduced, and besides that, the operator is prevented from forgetting about obtaining such information when an examination is conducted.

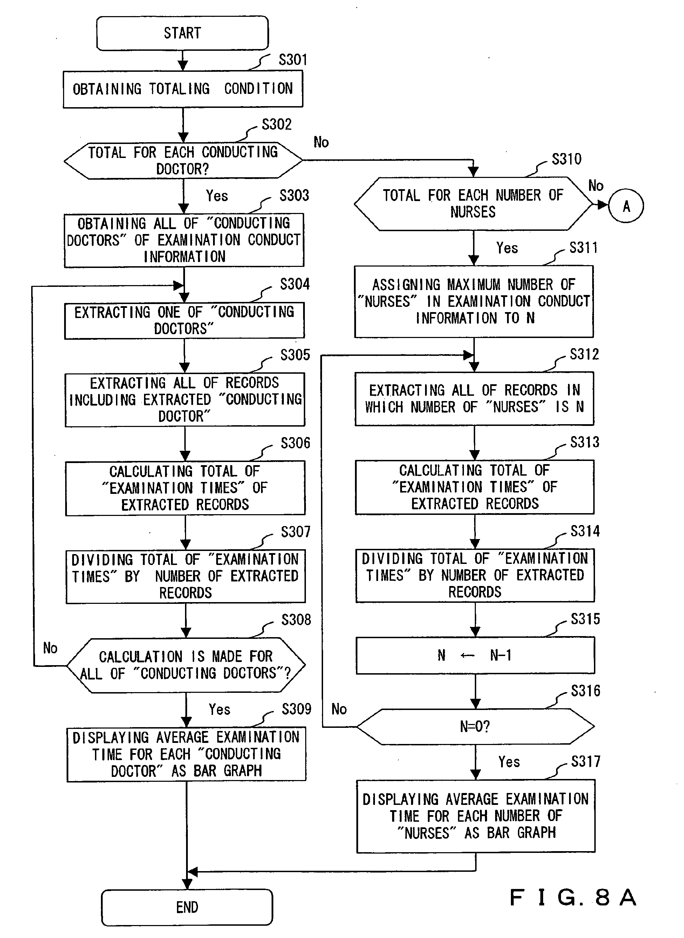

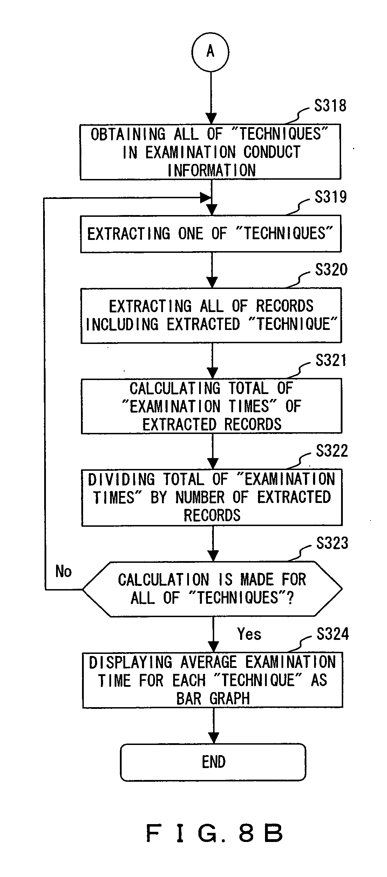

Next, Figs. 8A and 8B are explained. These figures depict the flowcharts representing the contents of the process for calculating a total of the examination conduct information 13-1 stored in the data server 13. This process is a process performed in the operations in S109 and S110 in the work flow shown in Fig. 5. Process steps, which are not particularly noted in the subsequent explanation, among respective steps shown in this figure, are performed by the CPU 21 of the examination information inputting/searching terminal 11-1. The CPU 21 implements these processes by reading and executing the control program stored in the storing unit 24 of the examination information inputting/searching terminal 11-1.

The totaling process shown in Figs. 8A and 8B is started when an instruction to calculate a total of the examination conduct information 13-1, which is issued with an operation for the inputting unit 25 of the examination information inputting/searching terminal 11-1, is detected.

Firstly, in S301 of Fig. 8A, a condition of the totaling made with the operation for the inputting unit 25 is obtained. This totaling condition is intended to specify any of an output of an average value of a time required for an examination, which is obtained with the total, for each doctor who conducts an examination, an output of the average value for each number of nurses who assists in the examination, or an output of the average value for each contents of a technique of the examination.

In S302, it is determined whether or not the totaling condition obtained with the process of the preceding step indicates the output for each doctor who conducts an examination. If a result of this determination is "Yes", the process proceeds to S303. If the result of this determination is "No", the process proceeds to S310.

In S303, all of files of the examination conduct information 13-1 in the data server 13 are referenced, and the data server 13 is made to perform a process for obtaining all of doctor names indicated in the "conducting doctor" field in at least any one of the records. This process is started when an instruction to execute the process is addressed and transmitted from the examination information inputting/searching terminal 11-1 to the data server 13, and reception of this instruction is detected by the CPU 21 of the data server 13, which is executing the control program. Thereafter, upon termination of this process, the obtained doctor names are transmitted from the data server 13 to the examination information inputting/searching terminal 11-1.

In S304, one of the doctor names obtained with the process of S303 is extracted.

In S305, a process for extracting a record, in which the doctor name extracted with the process of the preceding step is indicated in the "conducting doctor" field, from all of the files of the examination conduct information 13-1 in the data server 13 is performed. In the following S306, a process for calculating a time required for the examination with a technique indicated by the extracted record from times indicated by the "start time" field and the "end time" field of the record, and for calculating a total of that examination time in all of records is performed. In the further following S307, a process for obtaining an average of times required for the examination by dividing the totaled time required for the examination by the number of records extracted with the process of S305 is performed.

A series of these processes in S305 to S307 is performed when an instruction to perform a corresponding process is addressed and transmitted from the examination information inputting/searching terminal 11-1 to the data server 13, and reception of this instruction is detected by the CPU 21 of the data server 13, which is executing the control program. Thereafter, upon termination of this process, the obtained average time required for the examination is transmitted from the data server 13 to the examination information inputting/searching terminal 11-1.

In S308, it is determined whether or not the processes in S304 to S307 are performed for all of the doctor names obtained with the process of S303, and an average time required for the examination is obtained. If a result of this determination is "Yes", the process proceeds to S309. On the other hand, if the result of this determination in S308 is "No", the process returns to S304, and the processes in and after S304 are performed for a "conducting doctor" whose average time required for the examination is yet to be obtained.

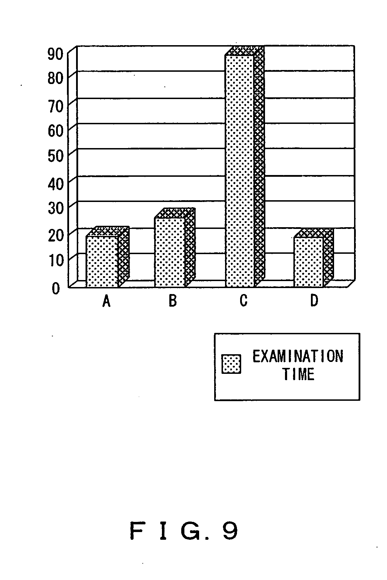

In S309, a process for plotting a bar graph that takes doctor names obtained with the process of S303 as a horizontal axis, and average times required for an examination as a vertical axis, for example, in units of minutes on the displaying unit 26 is performed. An example of thus plotted graph that represents the examination times against the respective doctors is shown in Fig. 9. By referencing this graph, a predication of a time required for an examination, which is expected according to a doctor who is responsible for an examination newly reserved, can be made.

After the process of S309 is terminated, this totaling process is completed. If the result of the above described determination in S302 is "No", it is determined in S310 whether or not the totaling condition obtained with the process of S301 indicates the output for each number of nurses who assist in the examination. If a result of this determination is "Yes", the process proceeds to S310. If the result of this determination is "No", the totaling condition obtained with the process of S301 is regarded as indicating the output for each contents of an examination technique, and the process proceeds to S318 of Fig. 8B.

In S311, all of the files of the examination conduct information 13-1 in the data server 13 are referenced, the number of nurses whose names are indicated in the "nurse" field is examined for each record, and the maximum number of nurses is assigned to a variable N.

In S312, a process for causing the data server 13 to extract a record, in which names of the nurses the number of which is the variable N are indicated by the "nurse" field, from all of the files of the examination conduct information 13-1 in the data server 13 is performed. In the following S313, a process for calculating a time required for the examination with a technique indicated by the extracted record from the times indicated by the "start time" field and the "end time" field of the record, and for calculating a total of all of the records of that examination time is performed. In S314, a process for obtaining an average of the times required for the examination by dividing the totaled time required for the examination by the number of records extracted with the process of S312 is performed.

A series of these processes in S312 to S314 is performed when an instruction to perform a corresponding process is addressed and transmitted from the examination information inputting/searching terminal 11-1 to the data server 13, and reception of this instruction is detected by the CPU 21 of the data server 13, which is executing the control program. When these processes are terminated, the obtained average time required for the examination is transmitted from the data server 13 to the examination information inputting/searching terminal 11-1.

In S315, a process for decrementing the value of the variable N by 1 is executed. In the following S316, it is determined whether or not the value of the variable N reaches 0. Here, if a result of this determination is "Yes", the totaling for each number of nurses is regarded as being completed, and the process proceeds to S317. If the result of this determination is "No", the process returns to S312, and the above described processes are repeated.

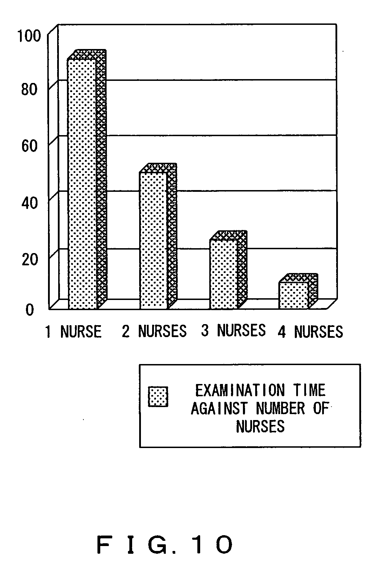

In S317, a process for plotting a bar graph that takes the numbers of nurses who assist in conducting the examination as a horizontal axis, and average times required for the examination as a vertical axis, for example, in units of minutes on the displaying unit 26 is performed. An example of thus plotted graph which represents the examination times against the respective numbers of nurses is shown in Fig. 10. By referencing this graph, a predication of a time required for an examination, which is expected according to the number of nurses who join as assisting persons when an examination newly reserved is conducted, can be made.

After the process of S317 is terminated, this totaling process is completed.

On the other hand, if the result of the above described determination in S310 is "No", and if the totaling condition obtained with the process of S301 is regarded as indicating the output for each contents of an examination technique, the process transfers to Fig. 8B. In S318, all of the files of the examination conduct information 13-1 in the data server 13 are referenced, and the data server 13 is made to perform a process for obtaining all of contents of an examination technique indicated by the "technique" field of at least one of records. This process is started when an instruction to perform a corresponding process is addressed and transmitted from the examination information inputting/searching terminal 11-1 to the data server 13, and reception of this instruction is detected by the CPU 21 of the data server 13, which is executing the control program. Thereafter, upon termination of this process, the obtained contents of the examination technique is transmitted from the data server 13 to the examination information inputting/searching terminal 11-1.

In S319, one of the contents of techniques obtained with the process of S318 is extracted.

In S320, a process for extracting a record, in which the contents of the examination technique extracted with the process of the preceding step is indicated in the "technique" field, from all of the files of the examination conduct information 13-1 in the data server 13 is performed. In the following S321, a process for calculating a time required for the examination with the examination technique indicated by the extracted record from the times indicated by the "start time" field and the "end time" field of the record, and for calculating a total of all of records of that examination time is performed. In S322, a process for obtaining an average amount of time required for the examination by dividing the totaled time required for the examination by the number of records extracted with the process of S320 is performed.

A series of these processes in S320 to S322 is performed when an instruction to perform a corresponding process is addressed and transmitted from the examination information inputting/searching terminal 11-1 to the data server 13, and reception of this instruction is detected by the CPU 21 of the data server 13, which is executing the control program. Thereafter, upon termination of this process, the obtained average time required for the examination is transmitted from the data server 13 to the examination information inputting/searching terminal 11-1.

In S323, it is determined whether or not the processes in S319 to S322 are performed for all of the contents of examination techniques obtained with the process of S318, and an average time required for the examination is obtained. If a result of this determination is "Yes", the process proceeds to S324. On the other hand, if the result of this determination in S323 is "No", the process returns to S319, and the processes in and after S319 are performed for a technique whose average time required for an examination is yet to be obtained.

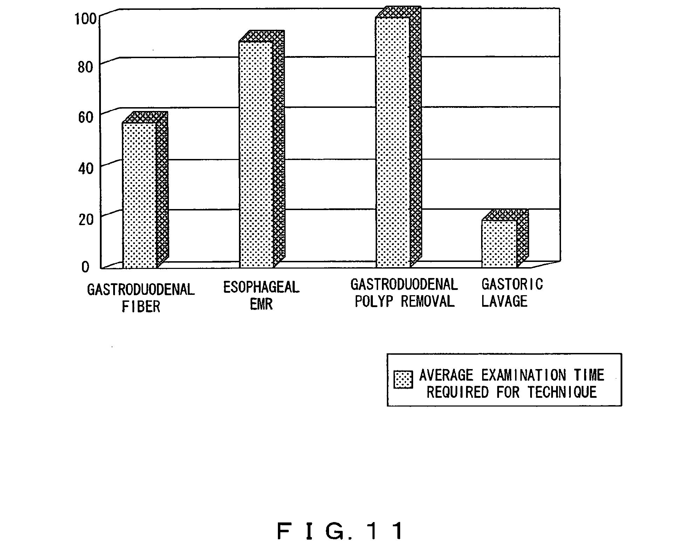

In S324, a process for plotting a bar graph that takes the contents of the examination techniques obtained with the process of S318 as a horizontal axis, and average times required for the examination as a vertical axis, for example, in units of minutes on the displaying unit 26 is performed. An example of thus plotted graph that represents the examination times against the respective examination techniques is shown in Fig. 11. By referencing this graph, a predication of a time required for an examination, which is expected according to the contents of a technique of an examination newly reserved, can be made.

After the process of S324 is terminated, this totaling process is completed.

The process up to this point is the totaling process.

In this totaling process, a name of a conducting doctor and the contents of an examination technique are obtained from the examination conduct information 13-1 with the process of S303 or S318. However, a table that indicates a name of a conducting doctor, and the contents of an examination technique is sometimes prepared beforehand in the data server 13, the above described HIS, or the like. Therefore, these information items may be obtained from these tables in that case.

Additionally, in this totaling process, the output for each number of nurses who assist in an examination is selected as a totaling condition. However, the output for each nurse who assists in an examination may be selected as a totaling condition. As a process in this case, a process similar to that in the case where the output for each doctor who conducts an examination is selected as a totaling condition may be performed.

Furthermore, in this totaling process, one of the three types of outputs such as the output for each doctor who conducts an examination, the output for each number of nurses who assist in an examination, and the output for each contents of an examination technique is selected as a totaling condition. However, a combination of these outputs can be also set as a totaling condition. To implement this, a record that meets a combined totaling condition may be extracted in the process of S305 or S312 of Fig. 8A, or S320 of Fig. 8B, in which records that meet a totaling condition are extracted from all of files of the examination conduct information 13-1. By doing this, it can be anticipated that a prediction of a required time, which is expected for an examination newly reserved, is more enhanced.

Still further, in this totaling process, a display of an average time required for an examination on the displaying unit 26 under each totaling condition is made by plotting a bar graph with the process of S309, S317, or S324. However, other types of graphs such as a line graph, a circle graph, etc. may be plotted instead of a bar graph, and besides, an average time required for an examination may be displayed directly with a numerical value as a matter of course. In addition, a process for calculating each average time required for an examination for all of the three types of the above described totaling conditions may be performed, and their graphs may be arranged and displayed on the same screen of the displaying unit 26.

At the same time, the present invention can be also implemented by creating a program for causing a computer having a standard configuration to perform the above described registration process and totaling process, and by causing such a computer to execute this program. Additionally, the present invention can be also implemented by recording such a program on a computer-readable storage medium, by causing a computer to read the program from the storage medium, and by causing a CPU comprised by the computer to execute the program.

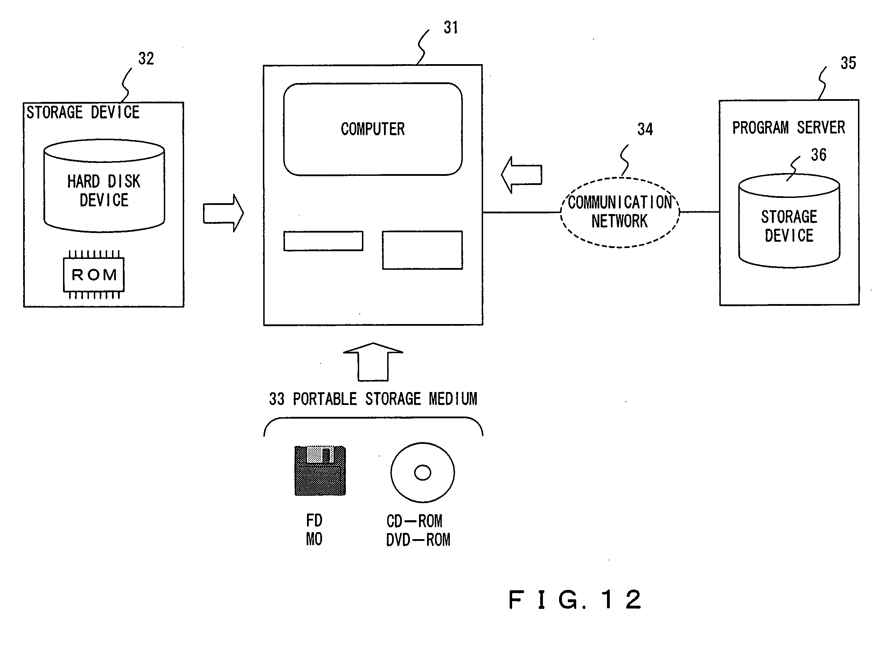

A storage medium from which a recorded control program can be read by a computer is exemplified in Fig. 12. As such a storage medium, for example, a storage device 32 such as a ROM, a hard disk device, etc., which is comprised as an included/externally attached device in/to the computer 31, a portable storage medium 33 etc., such as an FD (Flexible Disk), an MO (Magneto-Optical disk), a CD-ROM, a DVD-ROM, etc., are available.

Additionally, a storage device 36, which is included/externally attached in/to a program server 35 connected to the computer 31 via a communications network 34, may be a storage medium. In such a case, a transmission signal obtained by modulating a carrier wave with a control program recorded on the storage device 36 on the program server 35 side may be transmitted from the program server 35 side, and the control program may be demodulated from the transmission signal received via the communications network 34, and the CPU may be made to execute the control program on the computer 31 side.

Besides, the present invention is not limited to the above described preferred embodiment, and various improvements and modifications can be made.

As described above in detail, according to the present invention, examination conduct information, which occurs by conducting an examination in a medical institution, is stored in a storing unit each time the examination is conducted, and total amount of time required for conducting the examination, which is indicated by the examination conduct information stored in the storing unit, is calculated.

By doing so, a prediction of a required time, which is expected for a newly reserved examination, can be made by using a result of the total amount of time required for the examination conducted in the past.

## Claims

1. A system assisting in a medical service, comprising:
an examination conduct information storing unit (1,13) storing examination conduct information, which is information occurring by conducting an examination in a medial institution, each time the examination is conducted; and
a totaling unit (2,11-1) calculating a total amount of time required for conducting the examination, which is indicated by the examination conduct information stored in the examination conduct information storing unit.

2. The system according to claim 1, further comprising:
an obtaining unit (25) obtaining an input of the examination conduct information; and
a displaying unit (26) displaying a result of the total.

3. The system according to claim 1, wherein
the total is a total for each doctor who is responsible for the examination, for each number of assisting persons who assist in the examination, or for each examination technique in the examination.

4. The system according to claim 1, wherein
the totaling unit calculates an average of required times as a result of the total.

5. The system according to claim 4, further comprising
a display controlling unit graphing the result of the total, and causing the displaying unit to display the result.

6. A method assisting in a medical service, comprising:
storing examination conduct information, which is information occurring by conducting an examination in a medical institution, each time the examination is conducted; and
calculating a total amount of time required for conducting the examination, which is indicated by the stored examination conduct information.

7. A storage medium on which is recorded a program for causing a computer to assist in a medical service, and from which the program can be read by the computer, the program, by being executed by the computer, causing the computer to perform:
storing examination conduct information, which is information occurring by conducting an examination in a medical institution, in a storing unit, each time the examination is conducted; and
calculating a total amount of time required for conducting the examination, which is indicated by the examination conduct information stored in the storing unit.

8. A system assisting in a medical service, comprising:
examination conduct information storing means for storing examination conduct information, which is information occurring by conducting an examination in a medical institution, each time the examination is conducted; and
totaling means for calculating a total amount of time required for conducting the examination, which is indicated by the examination conduct information stored in the examination conduct information storing unit.
